# EUROPEAN PATENT APPLICATION

(11) **EP 4 813 406 A1**
(43) Date of publication of application: **30.09.2026**
(21) Application number: 24893001.8
(22) Date of filing: 12.09.2024
(51) Int. Cl.: A61K 39/12, A61K 39/155, A61P 31/14, C07K 14/08, C07K 14/115

(54) **MRNA VACCINE AND USE THEREOF**

(30) Priority: 22.11.2023 CN 202311566372
(71) Applicant: Changchun Bcht Biotechnology Co., Jilin 130012 (CN)
(72) Inventor: TANG, Xin, Changchun, Jilin 130012 (CN); ZANG, Yang, Changchun, Jilin 130012 (CN); SONG, Yueshuang, Changchun, Jilin 130012 (CN); DING, Nan, Changchun, Jilin 130012 (CN); LIU, Dawei, Changchun, Jilin 130012 (CN); ZHANG, Haiping, Changchun, Jilin 130012 (CN)
(74) Representative: Gill Jennings & Every LLP
(86) International application number: PCT/CN2024/118462
(87) International publication number: WO 2025/107842

(57) **Abstract**

Provided are an mRNA vaccine and use thereof. The mRNA vaccine comprises nucleic acids encoding a fusion F protein of respiratory syncytial virus and at least one matrix protein (M) of metapneumovirus. The mRNA vaccine can induce animals to generate specific humoral immune responses (neutralizing antibodies) against respiratory syncytial virus (RSV), can protect the animals from death caused by RSV infection, and reduces the viral load after challenge.

## Description

### TECHNICAL FIELD

The present invention relates to the field of biomedicines, and in particular to an mRNA vaccine and use thereof.

### BACKGROUND ART

Respiratory syncytial virus (RSV) is a common cause of acute respiratory diseases. RSV infection may occur in people of all ages, and can be life-threatening in infants, the elderly, and the immunocompromised adults. Global epidemiological studies have shown that 2-5% of the children infected with RSV require hospitalization. RSV disease causes 100,000 to 200,000 deaths per year globally. A key contributor to this enormous disease burden is the limited options of the existing prophylactic and therapeutic schemes.

RSV is an enveloped, single-stranded, negative-sense non-segmented RNA virus that has been classified as a member of the *Pneumovirus* genus within the *Paramyxoviridae* family. There are two major glycoproteins: adhesion protein (RSV G) and fusion protein (RSV F) on the surface of RSV. RSV G enhances *in-vivo* pathogenicity and promotes adhesion to host cells. RSV F is a class I fusion protein that mediates the fusion of the viral membrane with a host cell membrane and is a major target for inducing the human immune system to produce neutralizing antibodies.

The F protein has two conformations: prior to the infection of a human cell, it is in the prefusion conformation. The F protein is present in a trimeric form and contains a major antigenic site Ø, which is a major target of neutralizing antibodies *in vivo*. After binding to a receptor on the surface of a host cell, the F protein will undergo an irreversible conformational change, i.e., post-fusion conformation. This change allows it to insert into a host cell membrane and leads to the fusion of the virus with the host cell membrane, while the major target of neutralizing antibodies disappears. If the immune system encounters the F protein in its pre-fusion conformation, it can produce potent neutralizing antibodies. However, if the F protein is in its post-fusion conformation, it can hardly stimulate the production of neutralizing antibodies with high neutralizing titers. Although about 50% of the surface is shared by the pre-fusion and post-fusion conformations, the antigenic sites that are most sensitive to neutralization are located only on the pre-fusion conformation. Therefore, it is of paramount importance to design an antigen for the pre-fusion conformation of the F protein.

Studies have shown that the RSV F protein polymer precursor is post-translationally activated in the Golgi apparatus by furin protease cleavages at sites I and II. The cleavage process generates two subunits, F1 and F2, joined together by two disulfide cysteine-cysteine bridges, and releases a short 27 amino-acid glycopeptide called p27. Thus, the N-terminus of the F1 subunit exposes the hydrophobic fusion peptide (FP), which triggers virion-cell fusion following its insertion in the target membrane. The C-terminus of RSV F1 contains a cytosolic tail (CT) domain that interacts with a matrix protein (M) during virion assembly.

Currently, several antibody drugs (such as Palivizumab) have been developed, but they are limited for prophylactic use only in high-risk new-born infants. This limitation is directly caused by the low neutralizing potency of the developed RSV antibodies, and thus multiple high-dose administrations are required.

Therefore, it is absolutely necessary to develop an efficient and safe RSV vaccine.

### SUMMARY OF THE INVENTION

In view of the above, the object of the present invention is to provide an mRNA vaccine and use thereof. The mRNA vaccine can induce animals to generate specific humoral immune responses (neutralizing antibodies) against respiratory syncytial virus (RSV), can protect the animals from death caused by RSV infection, and reduces the viral load after challenge.

To achieve the above object, a first aspect of the present invention provides an mRNA vaccine comprising nucleic acids encoding a fusion F protein of respiratory syncytial virus and at least one matrix protein (M) of metapneumovirus.

In a preferred embodiment of the present invention, the fusion F protein of respiratory syncytial virus (RSV F) is a pre-fusion F protein of respiratory syncytial virus (RSV pre-F), and the at least one matrix protein (M) of metapneumovirus is one and/or two matrix proteins (M) of metapneumovirus;
preferably, the pre-fusion F protein of respiratory syncytial virus (RSV pre-F) is a pre-fusion F protein of human respiratory syncytial virus, and the matrix protein (M) of metapneumovirus (MPV) is a matrix protein (M) of human metapneumovirus (hMPV).

In a preferred embodiment of the present invention, the pre-fusion F protein of respiratory syncytial virus has the following amino acid sequence:
(1) the amino acid sequence as shown in SEQ ID NO: 1; or
(2) an amino acid sequence obtained from the amino acid sequence as shown in (1) by substituting, deleting or adding one or more residues, and having an identical or similar function to that of (1); or
(3) an amino acid sequence having at least 70% identity to the amino acid sequence as shown in (1) or (2).

In a preferred embodiment of the present invention, the matrix protein (M) of metapneumovirus has the following amino acid sequence:
(1) the amino acid sequence as shown in SEQ ID NO: 2; or
(2) an amino acid sequence obtained from the amino acid sequence as shown in (1) by substituting, deleting or adding one or more residues, and having an identical or similar function to that of (1); or
(3) an amino acid sequence having at least 70% identity to the amino acid sequence as shown in (1) or (2).

In a preferred embodiment of the present invention, one fusion F protein of respiratory syncytial virus is linked to at least one matrix protein (M) of metapneumovirus via a tandem gene expression element;
preferably, the nucleic acid encoding one pre-fusion F protein of respiratory syncytial virus and the nucleic acid encoding two matrix proteins (M) of metapneumovirus are operably linked to a tandem gene expression element, wherein the tandem gene expression element is an internal ribosome entry site (IRES);
more preferably, the nucleic acid sequence of the IRES is shown in SEQ ID NO: 3.

In a preferred embodiment of the present invention, the fusion F protein of respiratory syncytial virus comprises one or more amino acid residue modifications;
preferably, in the pre-fusion F protein of respiratory syncytial virus, compared with F protein of the natural respiratory syncytial virus, the wild-type amino acid residue(s) at one or more positions selected from positions 112, 155, 190, 207, 290, 379, and/or 447 are substituted with another amino acid residue(s).

In a preferred embodiment of the present invention, the transmembrane domain of the pre-fusion F protein of respiratory syncytial virus (RSV pre-F) is replaced with the transmembrane domain of the matrix protein (M) of metapneumovirus (MPV); and/or the cytosolic tail domain (CT) of the pre-fusion F protein of respiratory syncytial virus (RSV pre-F) is replaced with the cytosolic tail domain (CT) of the matrix protein (M) of metapneumovirus (MPV);
preferably, the transmembrane domain of the pre-fusion F protein of respiratory syncytial virus (RSV pre-F) is replaced with the transmembrane domain of the matrix protein (M) of human metapneumovirus (hMPV); and the cytosolic tail domain (CT) of the pre-fusion F protein of respiratory syncytial virus (RSV pre-F) is replaced with the cytosolic tail domain (CT) of the matrix protein (M) of human metapneumovirus (hMPV);
more preferably, the amino acid residues at positions 525-574 of the pre-fusion F protein of respiratory syncytial virus (RSV pre-F) are replaced with the amino acid residues at positions 489-539 of the matrix protein (M) of human metapneumovirus (hMPV).

In a preferred embodiment of the present invention, the mRNA vaccine has the following nucleic acid:
(4) the nucleotide sequence as shown in SEQ ID NO: 4, SEQ ID NO: 5 and/or SEQ ID NO: 6; or (5) a nucleotide sequence obtained from the nucleotide sequence as shown in (4) by substituting, deleting or adding one or more bases, and having an identical or similar function to that of (4); or (6) a nucleotide sequence having at least 70% identity to the nucleotide sequence as shown in (4) or (5).

A second aspect of the present invention provides use of the above mRNA vaccine in the manufacture of a respiratory syncytial virus vaccine.

A third aspect of the present invention provides a DNA construct comprising nucleic acid sequences encoding a fusion F protein of respiratory syncytial virus (RSV F) and a matrix protein (M) of metapneumovirus (MPV);
preferably, the fusion F protein of respiratory syncytial virus (RSV F) is a pre-fusion F protein of respiratory syncytial virus (RSV pre-F);
more preferably, the pre-fusion F protein of respiratory syncytial virus (RSV pre-F) is a pre-fusion F protein of human respiratory syncytial virus, and the matrix protein (M) of metapneumovirus is a matrix protein (M) of human metapneumovirus (hMPV).

In a preferred embodiment of the present invention, the transmembrane domain of the pre-fusion F protein of respiratory syncytial virus (RSV pre-F) is replaced with the transmembrane domain of the matrix protein (M) of metapneumovirus (MPV); and/or the cytosolic tail domain (CT) of the pre-fusion F protein of respiratory syncytial virus (RSV pre-F) is replaced with the cytosolic tail domain (CT) of the matrix protein (M) of metapneumovirus (MPV);
preferably, the transmembrane domain of the pre-fusion F protein of respiratory syncytial virus (RSV pre-F) is replaced with the transmembrane domain of the matrix protein (M) of human metapneumovirus (hMPV); and the cytosolic tail domain (CT) of the pre-fusion F protein of respiratory syncytial virus (RSV pre-F) is replaced with the cytosolic tail domain (CT) of the matrix protein (M) of human metapneumovirus (hMPV);
more preferably, the amino acid residues at positions 525-574 of the pre-fusion F protein of respiratory syncytial virus (RSV pre-F) are replaced with the amino acid residues at positions 489-539 of the matrix protein (M) of human metapneumovirus (hMPV).

The present invention achieves the following advantageous effects:
The mRNA vaccine of the present invention can induce animals to generate specific humoral immune responses (neutralizing antibodies) against respiratory syncytial virus (RSV), can protect the animals from death caused by RSV infection, and reduces the viral load after challenge, and thus has good application prospects.

### BRIEF DESCRIPTION OF THE DRAWINGS

FIG. 1 shows serum binding activity levels (FM-mRNA-1, FM-mRNA-2, FM-mRNA-3 and DS-Cav1 protein) in mice immunized with the mRNA vaccine as measured by ELISA assay;
FIG. 2 shows serum neutralizing activity levels (FM-mRNA-1, FM-mRNA-2, FM-mRNA-3 and DS-Cav1 protein) in mice immunized with the mRNA vaccine as measured by immunofluorescence assay;
FIG. 3 shows the pulmonary viral load results (FM-mRNA-1, FM-mRNA-2, FM-mRNA-3 and DS-Cav1 protein) in mice immunized with the mRNA vaccine after the challenge with RSV (LONG strain);
FIG. 4 shows serum binding activity levels (FM-mRNA-2, DS-Cav1 mRNA, DS2 mRNA and DS-Cav1 protein) in mice immunized with the mRNA vaccine as measured by ELISA assay;
FIG. 5 shows serum neutralizing activity levels (FM-mRNA-2, DS-Cav1 mRNA, DS2 mRNA and DS-Cav1 protein) in mice immunized with the mRNA vaccine as measured by immunofluorescence assay;
FIG. 6 shows the pulmonary viral load results (FM-mRNA-2, DS-Cav1 mRNA, DS2 mRNA and DS-Cav1 protein) in mice immunized with the mRNA vaccine after the challenge with RSV (LONG strain).

### DETAILED DESCRIPTION OF EMBODIMENTS

It should be noted that, unless otherwise defined, the technical terms or scientific terms used in the present application shall have the general meanings as understood by those skilled in the art to which the present disclosure pertains.

The experimental methods used in the following examples are conventional methods unless otherwise specified. The raw materials of medicine and reagent materials used in the following examples are all commercially available products unless otherwise specified.

When used in this application and the accompanying claims, the singular forms "a", "an", "another", and "the/said" include plural referents, unless the context explicitly indicates otherwise.

"Amino acid substitution/replacement": substitution/replacement of one amino acid in a polypeptide with a different amino acid or no amino acid (i.e., deletion).

Cytosol tail domain (CT): a contiguous region of a transmembrane protein that includes the protein's terminal (either N-terminus or C-terminus) and extends from the cytoplasmic surface of the cell membrane or viral envelope into the cytoplasm of the cell or enveloped virus. In the case of a type-I transmembrane protein, CT comprises the C-terminus of the protein. In the case of a type-II transmembrane protein, CT comprises the N-terminus of the protein.

Transmembrane domain (TM): an amino acid sequence of transmembrane lipid bilayer (such as lipid bilayer of a cell or virus). A transmembrane domain can be used for anchoring antigens to the membrane.

In the present invention, F protein of the natural respiratory syncytial virus refers to the pre-fusion and/or post-fusion F protein of respiratory syncytial virus.

"Sequence identity" between two polypeptide sequences or nucleic acid sequences refers to the percentage of identical residues between the sequences out of the total number of residues. When calculating the percentage of identity, the sequences being compared are aligned in a way to produce maximum matches between the sequences, and the gaps (if any) in the alignment are resolved by a specific algorithm. Preferred computer program methods for determining the identity between two sequences include, but are not limited to, the GCG program package, including GAP, BLASTP, BLASTN, and FASTA (Altschul et al., 1990, J. Mol. Biol. 215: 403-410). The above programs are publicly available from the National Center for Biotechnology Information (NCBI) and other sources. The well-known Smith-Waterman algorithm can also be used for determining identity.

The present invention provides an mRNA vaccine comprising nucleic acids encoding a fusion F protein of respiratory syncytial virus and at least one matrix protein (M) of metapneumovirus.

In the present invention, the mRNA vaccine comprises at least one of the following structural modifications:
1. replacing/substituting the amino acid residues in certain positions of the RSV F protein to maintain the RSV F protein in the pre-fusion conformation; for example, in the pre-fusion F protein of respiratory syncytial virus, compared with F protein of the natural respiratory syncytial virus, the wild-type amino acid residue(s) at one or more positions selected from positions 112, 155, 190, 207, 290, 379, and/or 447 are substituted with another amino acid residue(s);
2. replacing the transmembrane domain of the RSV F protein with the corresponding transmembrane domain of matrix protein (M) of metapneumovirus (MPV);
3. replacing the cytosolic tail domain of the pre-fusion F protein of respiratory syncytial virus (RSV pre-F) with the corresponding cytosolic tail domain of matrix protein (M) of metapneumovirus (MPV).

Preferably, the mRNA vaccine comprises a first structural modification and a second structural modification, or a first structural modification and a third structural modification, or simultaneously comprises a first structural modification, a second structural modification and a third structural modification.

In the present invention, the nucleic acid vaccine designed with such modifications has the ability to stimulate humoral and cellular immune responses *in vivo*.

The mRNA vaccine of the present invention is recombinantly engineered to co-express matrix protein (M protein) of metapneumovirus (MPV)and RSV glycoprotein (F protein). When the mRNA vaccine is administered to a subject, it can induce an immune response, including the induction of neutralizing antibodies. The mRNA vaccine can elicit higher immune protection and prevent the development of viral mutants that could evade the immune system.

In a preferred embodiment of the present invention, the pre-fusion F protein of respiratory syncytial virus has the following amino acid sequence:
(1) the amino acid sequence as shown in SEQ ID NO: 1; or
(2) an amino acid sequence obtained from the amino acid sequence as shown in (1) by substituting, deleting or adding one or more residues, and having an identical or similar function to that of (1); or
(3) an amino acid sequence having at least 70% identity to the amino acid sequence as shown in (1) or (2).

In a preferred embodiment of the present invention, the matrix protein (M) of metapneumovirus has the following amino acid sequence:
(1) the amino acid sequence as shown in SEQ ID NO: 2; or
(2) an amino acid sequence obtained from the amino acid sequence as shown in (1) by substituting, deleting or adding one or more residues, and having an identical or similar function to that of (1); or
(3) an amino acid sequence having at least 70% identity to the amino acid sequence as shown in (1) or (2).

The mRNA vaccine of the present invention simultaneously comprises an amino acid sequence of the fusion F protein of respiratory syncytial virus and an amino acid sequence of at least one matrix protein (M) of metapneumovirus, and a nucleic acid sequence of a tandem gene expression element;
preferably, the nucleic acid encoding one pre-fusion F protein of respiratory syncytial virus and the nucleic acid encoding two matrix proteins (M) of metapneumovirus are operably linked to a tandem gene expression element, wherein the tandem gene expression element is an internal ribosome entry site (IRES);
more preferably, the nucleic acid sequence of the IRES is shown in SEQ ID NO: 3.

In a preferred embodiment of the present invention, the mRNA vaccine has the following nucleic acid:
(4) the nucleotide sequence as shown in SEQ ID NO: 4, SEQ ID NO: 5 and/or SEQ ID NO: 6; or
(5) a nucleotide sequence obtained from the nucleotide sequence as shown in (4) by substituting, deleting or adding one or more bases, and having an identical or similar function to that of (4); in this case, the function of the nucleotide sequence of (5) being identical or similar to that of (4) means that the nucleotide sequence of (5) can achieve an identical or similar function as the nucleotide sequence shown in SEQ ID NO: 4, SEQ ID NO: 5 and/or SEQ ID NO: 6 in inducing animals to generate specific humoral immune responses (neutralizing antibodies) against RSV; or
(6) a nucleotide sequence having at least 70% identity to the nucleotide sequence as shown in (4) or (5).

The present invention further provides a DNA construct comprising nucleic acid sequences encoding a fusion F protein of respiratory syncytial virus (RSV F) and a matrix protein (M) of metapneumovirus (MPV);
preferably, the fusion F protein of respiratory syncytial virus (RSV F) is a pre-fusion F protein of respiratory syncytial virus (RSV pre-F);
more preferably, the pre-fusion F protein of respiratory syncytial virus (RSV pre-F) is a pre-fusion F protein of human respiratory syncytial virus, and the matrix protein (M) of metapneumovirus is a matrix protein (M) of human metapneumovirus (hMPV).

Introducing the DNA construct of the present invention into cells can direct expression of the fusion F protein of respiratory syncytial virus (RSV F) and the matrix protein (M) of metapneumovirus (MPV), thereby forming the mRNA vaccine of the present invention.

In a preferred embodiment of the present invention, the transmembrane domain of the pre-fusion F protein of respiratory syncytial virus (RSV pre-F) is replaced with the transmembrane domain of the matrix protein (M) of metapneumovirus (MPV); and/or the cytosolic tail domain (CT) of the pre-fusion F protein of respiratory syncytial virus (RSV pre-F) is replaced with the cytosolic tail domain (CT) of the matrix protein (M) of metapneumovirus (MPV);
preferably, the transmembrane domain of the pre-fusion F protein of respiratory syncytial virus (RSV pre-F) is replaced with the transmembrane domain of the matrix protein (M) of human metapneumovirus (hMPV); and the cytosolic tail domain (CT) of the pre-fusion F protein of respiratory syncytial virus (RSV pre-F) is replaced with the cytosolic tail domain (CT) of the matrix protein (M) of human metapneumovirus (hMPV);
more preferably, the amino acid residues at positions 525-574 of the pre-fusion F protein of respiratory syncytial virus (RSV pre-F) are replaced with the amino acid residues at positions 489-539 of the matrix protein (M) of human metapneumovirus (hMPV).

The present invention is further illustrated by the following examples, which are described for illustrative purposes only and are not intended to limit the scope of the invention. Any simple modification made to the present invention based on the spirit of the present invention is within its protection scope. With reference to the examples, the present invention is further described below:

### Example 1: Preparation of mRNA vaccine

The amino acid sequences and nucleic acid sequences corresponding to the mRNA vaccine are shown in SEQ ID NOs: 1-6. All the sequences were prepared by Hzymes Biotechnology Co., Ltd. For detailed operational steps, reference may be made to the literature: Cimica V, Hélène Boigard, Bhatia B, et al. Novel Respiratory Syncytial Virus-Like Particle Vaccine Composed of the Postfusion and Prefusion Conformations of the F Glycoprotein. [J]. Clinical & Vaccine Immunology Cvi, 2016, 23(6):451-459.

### Example 2: Immunization Schedule of mRNA vaccine

The following experimental group and control group were provided:
Experimental groups: female BALB/c mice aged 6-8 weeks (5 mice/group) were divided into groups immunized with mRNA vaccine at the doses of 1 µg/mouse, 5 µg/mouse, and 10 µg/mouse (FM-mRNA-1, FM-mRNA-2, and FM-mRNA-3; the nucleic acid sequences thereof were shown in SEQ ID NOs: 4-6, respectively, and were prepared by Hzymes Biotechnology Co., Ltd.), and groups immunized with DS-Cav1 protein at the doses of 1 µg/mouse, 5 µg/mouse, and 10 µg/mouse (following the protocol as described in the reference literature: Structure-Based Design of a Fusion Glycoprotein Vaccine for Respiratory Syncytial Virus[J]. Science, 2013, 342(6158):592-598.). The mice were immunized twice on Day 1 and Day 22, respectively. Serum samples were collected on Day 32 for testing. The mice were challenged with RSV (LONG strain, purchased from ATCC) (at the titer of 2*10⁶FFU/mouse) on Day 33, anesthetized (approximately for 2-5 minutes), and then were further challenged with 25 µL of virus via intranasal injection. Lung tissues were collected on Day 3 post-challenge (D36) and then ground. Subsequently, the supernatants were taken for evaluating protection against the viral challenge.

### Example 3: Serum binding activity assay (by ELISA assay) in mice immunized with mRNA vaccine

The plate was initially coated with RSV pre-F protein at a concentration of 2 µg/mL (100µL/well) at 2-8 °C overnight. The plate was washed 3 times with PBS-T solution (2 minutes/time), added with a blocking solution, and incubated at 37 °C for 1 hour. Subsequently, the plate was further washed 3 times with PBS-T solution (2 minutes/time). The serum in the first-well was diluted at a ratio of 1:100 with PBS. Serum samples were 5-fold serially diluted. The blank wells were added with the antibody dilution solution alone. 100µL/well of the serum samples were taken and added to the plate, and then incubated at 37 °C for 1 hour. The plate was washed 3 times with PBS-T solution, added with HRP-labeled goat anti-mouse IgG (H+L) secondary antibody, and then incubated at 37 °C for 1 hour. Tetramethylbenzidine (TMB) chromogenic solution (obtained by blending equal amounts of solutions A and B) was added to develop color at room temperature for 15 minutes in the dark. 2M H₂SO₄ was added to terminate the color development. Absorbance values were measured using a microplate reader.

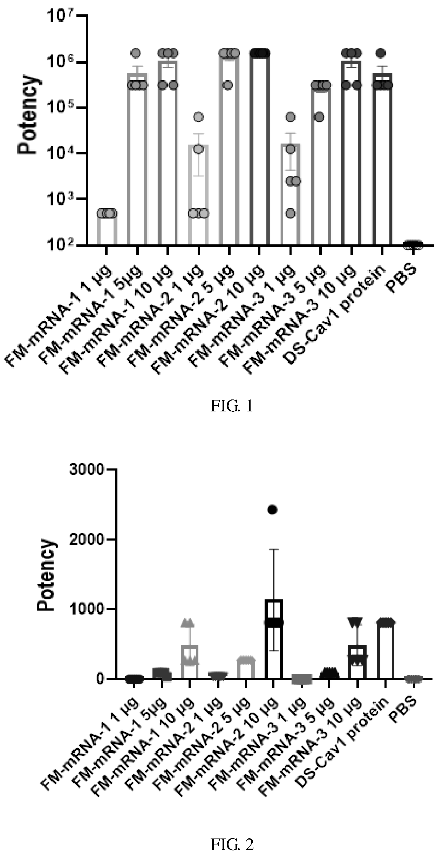

The experimental results were shown in FIG. 1: FM-mRNA-1, FM-mRNA-2 and FM-mRNA-3 used in different doses all showed significant immunological activity in dose-dependent manner. In particular, 10 µg/mL FM-mRNA-2 showed remarkably higher immunological effect than that of 10 µg/mL DS-Cav1 protein.

### Example 4: Serum neutralizing activity assay (by indirect immunofluorescence assay) in mice immunized with mRNA vaccine

Neutralizing antibodies against RSV in the sera of BALB/c mice immunized with the mRNA vaccine were measured using an indirect immunofluorescence assay. Synagis (purchased from Immunity Pharma) was diluted to 50 µg/mL in DMEM/F12 medium as a primary-antibody positive control. The 10-fold diluted serum samples to be tested and the positive control antibody Synagis were incubated with RSV (LONG strain) (titer: 2*10⁸FFU/mL, 1.0mL/vial, purchased from ATCC) for neutralization for 1 hour. HEp-2 cells (at a density of 1.2*10⁶/mL) were added and incubated in the incubator at 37 °C for 24 hours. Cells without adding virus were used as the negative control. After 24 hours, the supernatant was discarded, and added with 80% cold acetone to immobilized the cells at 4 °C. After 30 minutes, the acetone was discarded and dried. The primary antibody Synagis (1 mg/mL) was diluted to 1: 1000 (50 µL/well), and incubated at 37 °C for 1 hour. Subsequently, the supernatant was discarded. The plate was washed 3 times with PBS, added with Goat anti-human IgG-FITC (1: 400 dilution, 50 µL/well, purchased from Southern Biotech) and incubated at 37 °C for 1 hour. The plate was further washed 3 times with PBS. Percentage of fluorescent foci was observed under a fluorescence microscope.

The experimental results were shown in FIG. 2: cells in the negative control showed no fluorescence; RSV (LONG strain): the virus-positive cells showed 80%-95% of fluorescent foci. FM-mRNA-1, FM-mRNA-2 and FM-mRNA-3 used in the doses of 5 µg/mL and 10 µg/mL all showed significant immunological activity in dose-dependent manner. In particular, 10 µg/mL FM-mRNA-1 and 10 µg/mL FM-mRNA-3 had comparable immunological effect to that of 10 µg/mL DS-Cav1 protein, while 10 µg/mL FM-mRNA-2 showed significantly higher immunological effect than that of 10 µg/mL DS-Cav1 protein.

### Example 5: Evaluation of protection against viral challenge in mice immunized with mRNA vaccine

After lung tissues were ground, the supernatant was taken for pulmonary viral load detection (qPCR). During the experiment, hair loss of mice after immunization was observed and recorded. RNA was extracted according to the instruction manual of the Viral RNA Kit (purchased from Beijing TransGen Biotech Co., Ltd.), and then detected.

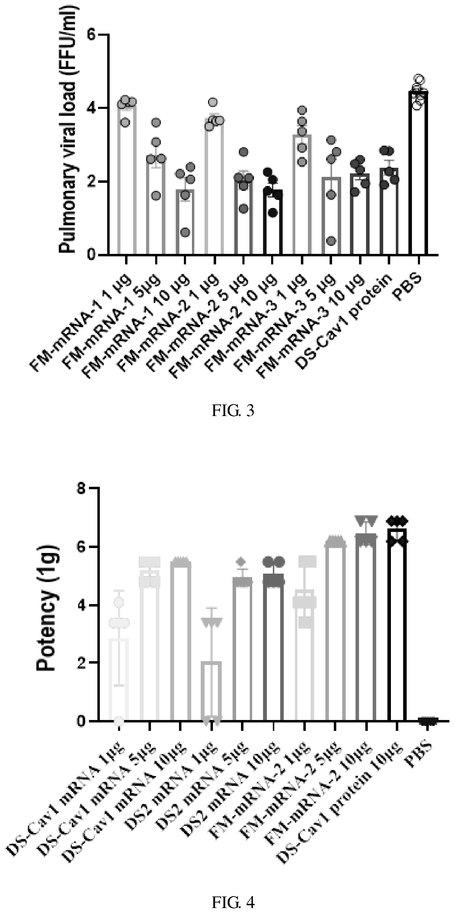

The experimental results were shown in FIG. 3: FM-mRNA-1, FM-mRNA-2 and FM-mRNA-3 used in different doses can protect animals from death caused by lethal RSV infection and reduce the pulmonary viral load after challenge. In particular, 10 µg/mL FM-mRNA-2 achieved the best effect.

The above examples indicated that FM-mRNA-1, FM-mRNA-2, and FM-mRNA-3 used in different doses all had remarkable binding activity and can induce animals to generate specific humoral immune responses (neutralizing antibodies) against RSV. Each mRNA vaccine can protect animals from death caused by lethal RSV infection and reduce the pulmonary viral load after challenge. The relationship of the immunogenicity and protection levels among the same dose groups is: FM-mRNA-2 ≥ DS-Cav1 protein > FM-mRNA-1 = FM-mRNA-3.

### Example 6: Immunization Schedule of mRNA vaccine

The following experimental group and control group were provided:
Experimental groups: female BALB/c mice aged 6-8 weeks (5 mice/group) were divided into groups immunized with mRNA vaccine at the doses of 1 µg/mouse, 5 µg/mouse, and 10 µg/mouse (FM-mRNA-2 was prepared by Hzymes Biotechnology Co., Ltd.), and groups immunized with DS-Cav1 mRNA, DS2 mRNA and DS-Cav1 protein (DS-Cav1 mRNA: reference may be made to the literature: Structure-Based Design of a Fusion Glycoprotein Vaccine for Respiratory Syncytial Virus[J]. Science, 2013, 342(6158):592-598. DS2 mRNA: reference may be made to the literature: Joyce M G, Zhang B, Ou L, et al. Iterative structure-based improvement of a respiratory syncytial virus fusion glycoprotein vaccine[J]. Nature Structural & Molecular Biology, 2016, 23(9):811-820. and DS-Cav1 protein, which were prepared by GenScript Biotech Corporation). The mice were immunized twice on Day 1 and Day 22, respectively. Serum samples were collected on Day 36 for testing. Blood was collected from the parotid gland and placed in a 1.5 ml EP tube. The serum was separated by centrifugation at 13,000 rpm for 10 minutes. The mice were challenged with RSV (LONG strain) (at the titer of 2*10⁶FFU/mouse) on Day 39, anesthetized (approximately for 2-5 minutes), and then were further challenged with 25 µL of virus via intranasal injection into each nose. Lung tissues were collected on Day 3 post-challenge (D42) and then ground. Subsequently, the supernatants were taken for evaluating protection against the viral challenge.

### Example 7: Serum binding activity assay (by ELISA assay) in mice immunized with mRNA vaccine

The plate was initially coated with RSV pre-F protein at a concentration of 2 µg/mL (100µL/well) at 2-8 °C overnight. The plate was washed 3 times with PBS-T solution (2 minutes/time), added with a blocking solution, and incubated at 37 °C for 1 hour. Subsequently, the plate was further washed 3 times with PBS-T solution (2 minutes/time). The serum in the first-well was diluted at a ratio of 1:500 with PBS. Serum samples were 5-fold serially diluted. The blank wells were added with the antibody dilution solution alone. 100µL/well of the serum samples were taken and added to the plate, and then incubated at 37 °C for 1 hour. The plate was washed 3 times with PBS-T solution, added with HRP-labeled goat anti-mouse IgG (H+L) secondary antibody, and then incubated at 37 °C for 1 hour. Tetramethylbenzidine (TMB) chromogenic solution (obtained by blending equal amounts of solutions A and B) was added to develop color at room temperature for 15 minutes in the dark. 2M H₂SO₄ was added to terminate the color development. Absorbance values were measured using a microplate reader.

The experimental results were shown in FIG. 4: FM-mRNA-2, DS-Cav1 mRNA, DS2 mRNA and DS-Cav1 protein in the doses of 1 µg/mouse, 5 µg/mouse and 10 µg/mouse all showed significant immunological activity. In particular, 10 µg/mL FM-mRNA-2 showed comparable immunological effect to that of 10 µg/mL DS-Cav1 protein.

### Example 8: Serum neutralizing activity assay (by indirect immunofluorescence assay) in mice immunized with mRNA vaccine

Neutralizing antibodies against RSV in the sera of BALB/c mice immunized with the mRNA vaccine were measured using an indirect immunofluorescence assay. Synagis (purchased from Immunity Pharma) was diluted to 50 µg/mL in DMEM/F12 medium as a primary-antibody positive control. The 10-fold diluted serum samples to be tested and the positive control antibody Synagis were incubated with RSV (LONG strain) (titer: 2*10⁸FFU/mL, 1.0mL/vial, purchased from ATCC) for neutralization for 1 hour. HEp-2 cells (at a density of 1.2*10⁶/mL) were added and incubated in the incubator at 37 °C for 24 hours. Cells without adding virus were used as the negative control. After 24 hours, the supernatant was discarded, and added with 80% cold acetone to immobilized the cells at 4 °C. After 30 minutes, the acetone was discarded and dried. The primary antibody Synagis (1 mg/mL) was diluted to 1: 1000 (50 µL/well), and incubated at 37 °C for 1 hour. Subsequently, the supernatant was discarded. The plate was washed 3 times with PBS, added with Goat anti-human IgG-FITC (1: 400 dilution, 50 µL/well, purchased from Southern Biotech) and incubated at 37 °C for 1 hour. The plate was further washed 3 times with PBS. Percentage of fluorescent foci was observed under a fluorescence microscope.

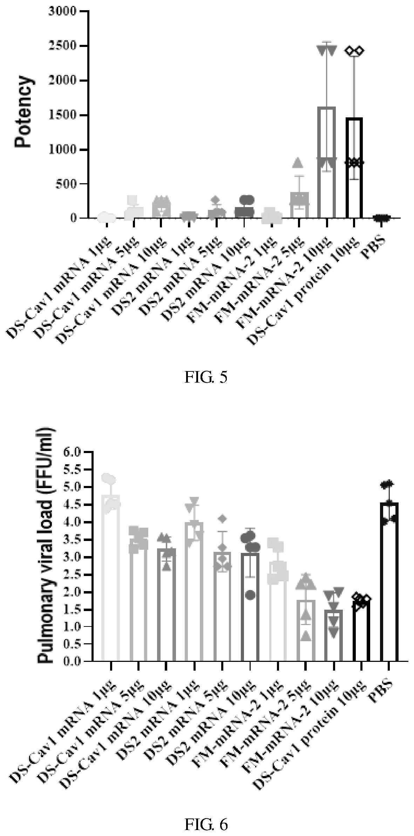

The experimental results were shown in FIG. 5: cells in the negative control showed no fluorescence; RSV (LONG strain): the virus-positive cells showed 80%-95% of fluorescent foci. After the second immunization, 10 µg/mL FM-mRNA-2 can produce remarkably higher levels of neutralizing antibodies than that of 10 µg/mL DS-Cav1 protein.

### Example 9: Evaluation of protection against viral challenge in mice immunized with mRNA vaccine

After lung tissues were ground, the supernatant was taken for pulmonary viral load detection (qPCR). During the experiment, hair loss of mice after immunization was observed and recorded. RNA was extracted according to the instruction manual of the Viral RNA Kit (purchased from Beijing TransGen Biotech Co., Ltd.), and then detected.

The experimental results were shown in FIG. 6: FM-mRNA-2, DS-Cav1 mRNA, DS2 mRNA and DS-Cav1 protein used in different doses can protect animals from death caused by lethal RSV infection and reduce the pulmonary viral load after challenge. 10 µg/mL FM-mRNA-2 resulted in lower pulmonary viral load after challenge than that of 10 µg/mL DS-Cav1 protein.

The above results of the examples indicated that FM-mRNA-2, DS-Cav1 mRNA, DS2 mRNA and DS-Cav1 protein used in different doses all had remarkable binding activity and can induce animals to generate specific humoral immune responses (neutralizing antibodies) against RSV. Each mRNA vaccine can protect animals from death caused by lethal RSV infection and reduce the pulmonary viral load after challenge. The relationship of the immunogenicity and protection levels among the same dose groups is: FM-mRNA-2 > DS-Cav1 protein > DS2 mRNA > DS-Cav1 mRNA.

Although the above examples have only described particular embodiments of the present invention, those skilled in the art could understand that these embodiments are described for illustrative purpose only, and the protection scope of the present invention is defined by the appended claims. Those skilled in the art may make various changes or modifications to these embodiments without departing from the principles and spirit of the present invention, and all such changes or modifications should fall within the protection scope of the present invention.

## Claims

1. An mRNA vaccine comprising nucleic acids encoding a fusion F protein of respiratory syncytial virus and at least one matrix protein (M) of metapneumovirus.

2. The mRNA vaccine according to claim 1, wherein the fusion F protein of respiratory syncytial virus is a pre-fusion F protein of respiratory syncytial virus, and the at least one matrix protein (M) of metapneumovirus is one and/or two matrix proteins (M) of metapneumovirus;
preferably, the pre-fusion F protein of respiratory syncytial virus is a pre-fusion F protein of human respiratory syncytial virus, and the matrix protein (M) of metapneumovirus is a matrix protein (M) of human metapneumovirus.

3. The mRNA vaccine according to claim 1 or 2, wherein the pre-fusion F protein of respiratory syncytial virus has the following amino acid sequence:
(1) the amino acid sequence as shown in SEQ ID NO: 1; or
(2) an amino acid sequence obtained from the amino acid sequence as shown in (1) by substituting, deleting or adding one or more residues, and having an identical or similar function to that of (1); or
(3) an amino acid sequence having at least 70% identity to the amino acid sequence as shown in (1) or (2).

4. The mRNA vaccine according to claim 1 or 2, wherein the matrix protein (M) of metapneumovirus has the following amino acid sequence:
(1) the amino acid sequence as shown in SEQ ID NO: 2; or
(2) an amino acid sequence obtained from the amino acid sequence as shown in (1) by substituting, deleting or adding one or more residues, and having an identical or similar function to that of (1); or
(3) an amino acid sequence having at least 70% identity to the amino acid sequence as shown in (1) or (2).

5. The mRNA vaccine according to any one of claims 1 to 4, wherein one fusion F protein of respiratory syncytial virus is linked to at least one matrix protein (M) of metapneumovirus via a tandem gene expression element;
preferably, the nucleic acid encoding one pre-fusion F protein of respiratory syncytial virus and the nucleic acid encoding two matrix proteins (M) of metapneumovirus are operably linked to a tandem gene expression element, wherein the tandem gene expression element is an internal ribosome entry site (IRES);
more preferably, the nucleic acid sequence of the IRES is shown in SEQ ID NO: 3.

6. The mRNA vaccine according to any one of claims 1 to 5, wherein the fusion F protein of respiratory syncytial virus comprises one or more amino acid residue modifications;
preferably, in the pre-fusion F protein of respiratory syncytial virus, compared with F protein of the natural respiratory syncytial virus, the wild-type amino acid residue(s) at one or more positions selected from positions 112, 155, 190, 207, 290, 379, and/or 447 are substituted with another amino acid residue(s).

7. The mRNA vaccine according to claim 6, wherein the transmembrane domain of the pre-fusion F protein of respiratory syncytial virus is replaced with the transmembrane domain of the matrix protein (M) of metapneumovirus; and/or the cytosolic tail domain of the pre-fusion F protein of respiratory syncytial virus is replaced with the cytosolic tail domain of the matrix protein (M) of metapneumovirus;
preferably, the transmembrane domain of the pre-fusion F protein of respiratory syncytial virus is replaced with the transmembrane domain of the matrix protein (M) of human metapneumovirus; and the cytosolic tail domain of the pre-fusion F protein of respiratory syncytial virus is replaced with the cytosolic tail domain of the matrix protein (M) of human metapneumovirus;
more preferably, the amino acid residues at positions 525-574 of the pre-fusion F protein of respiratory syncytial virus are replaced with the amino acid residues at positions 489-539 of the matrix protein (M) of human metapneumovirus.

8. The mRNA vaccine according to any one of claims 1 to 7, wherein the mRNA vaccine has the following nucleic acid:
(4) the nucleotide sequence as shown in SEQ ID NO: 4, SEQ ID NO: 5 and/or SEQ ID NO: 6; or
(5) a nucleotide sequence obtained from the nucleotide sequence as shown in (4) by substituting, deleting or adding one or more bases, and having an identical or similar function to that of (4); or
(6) a nucleotide sequence having at least 70% identity to the nucleotide sequence as shown in (4) or (5).

9. Use of the mRNA vaccine according to any one of claims 1 to 8 in the manufacture of a respiratory syncytial virus vaccine.

10. A DNA construct comprising nucleic acid sequences encoding a fusion F protein of respiratory syncytial virus and a matrix protein (M) of metapneumovirus;
preferably, the fusion F protein of respiratory syncytial virus is a pre-fusion F protein of respiratory syncytial virus;
more preferably, the pre-fusion F protein of respiratory syncytial virus is a pre-fusion F protein of human respiratory syncytial virus, and the matrix protein (M) of metapneumovirus is a matrix protein (M) of human metapneumovirus.

11. The DNA construct according to any one of claims 1 to 10, wherein the transmembrane domain of the pre-fusion F protein of respiratory syncytial virus is replaced with the transmembrane domain of the matrix protein (M) of metapneumovirus; and/or the cytosolic tail domain of the pre-fusion F protein of respiratory syncytial virus is replaced with the cytosolic tail domain of the matrix protein (M) of metapneumovirus;
preferably, the transmembrane domain of the pre-fusion F protein of respiratory syncytial virus is replaced with the transmembrane domain of the matrix protein (M) of human metapneumovirus; and the cytosolic tail domain of the pre-fusion F protein of respiratory syncytial virus is replaced with the cytosolic tail domain of the matrix protein (M) of human metapneumovirus;
more preferably, the amino acid residues at positions 525-574 of the pre-fusion F protein of respiratory syncytial virus are replaced with the amino acid residues at positions 489-539 of the matrix protein (M) of human metapneumovirus.
